# DEMANDE DE BREVET EUROPEEN

(11) **EP 0 730 142 A1**
(43) Date de publication de la demande: **04.09.1996**
(21) Numéro de dépôt: 96400429.5
(22) Date de dépôt: 29.02.1996
(51) Int. Cl.: G01N 1/00, G21F 7/047

(54) **Dispositif de prélèvement d'échantillons liquides**

(30) Priorité: 02.03.1995 FR 9502419
(71) Demandeur: COMPAGNIE GENERALE DES MATIERES NUCLEAIRES, F-78140 Velizy Villacoublay (FR)
(72) Inventeur: Coignaud, Gabriel, 78160 Marly-Le-Roi (FR); Richter, Roland, 78320 Le Mesnil-Saint-Denis (FR)
(74) Mandataire: Dubois-Chabert, Guy

(57) **Abrégé**

Un dispositif de prélèvement d'échantillons liquides comprend une aiguille (38) dont une extrémité basse débouche dans un réservoir (12) alimenté en liquide par un circuit en dépression. L'extrémité haute, pointue, de l'aiguille (38) est placée dans une boîte à gants d'un banc de prélèvement, de telle sorte qu'on peut enfoncer dessus un cruchon en dépression, pour y transférer un échantillon du liquide. Pour éviter que la pompe à vide du circuit n'agisse sur l'air contenu dans la boîte à gants, un manchon coulissant (48) recouvre normalement la partie haute de l'aiguille (38), dans laquelle le chas débouche latéralement.

## Description

L'invention concerne un dispositif de prélèvement d'échantillons liquides, comprenant une aiguille dont une extrémité d'aspiration débouche dans un réservoir susceptible d'être alimenté en liquide par un circuit en dépression et dont l'extrémité opposée est apte à perforer un cruchon en dépression, de telle sorte qu'un échantillon de liquide soit automatiquement prélevé dans le cruchon sous l'effet d'aspiration provoqué par la dépression régnant à l'intérieur de ce dernier.

Un tel dispositif de prélèvement d'échantillons liquides peut notamment être utilisé dans les industries nucléaire, chimique, pharmaceutique, etc.. Il permet de prélever des échantillons liquides d'une manière automatisée, ce qui le rend particulièrement adapté aux prélèvements de liquides dangereux pour l'homme et/ou l'environnement. Le conditionnement de l'échantillon liquide prélevé dans un cruchon permet d'effectuer ultérieurement sur cet échantillon toutes les mesures et analyses qui peuvent s'avérer nécessaires.

Dans les documents FR-A-1 401 298 et FR-A-2 058 751, on a décrit un banc de prise d'échantillons liquides comprenant plusieurs dispositifs de prélèvement dont les réservoirs sont alimentés par des circuits en dépression séparés, de telle sorte que les échantillons prélevés par chacun des dispositifs de prélèvement soient représentatifs de liquides situés en des emplacements différents d'une installation à surveiller. Les extrémités pointues des aiguilles de chacun des dispositifs de prélèvement débouchent dans une boîte à gants dans laquelle sont amenés les cruchons sous vide destinés à recevoir les échantillons. Cette boîte à gants est habituellement remplie d'air dont le renouvellement est assuré par une ventilation appropriée.

Par ailleurs, le document EP-A-0 296 917 décrit un circuit en dépression permettant d'acheminer un liquide situé en un emplacement donné d'une installation dans le réservoir d'un dispositif de prélèvement d'échantillons liquides analogue à ceux qui équipent le banc de prélèvement décrit dans les documents FR-A-1 401 298 et FR-A-2 058 751. Dans un tel circuit, l'alimentation du réservoir est assurée par une pompe à vide. Plus précisément, de l'air comprimé est mélangé au liquide en amont du dispositif de prélèvement, puis séparé du liquide en aval de ce dispositif, pour être aspiré par la pompe à vide.

Dans le document EP-A-0 078 211, on a décrit un dispositif de prélèvement d'échantillons liquides, ainsi qu'un banc de prélèvement équipé d'un grand nombre de dispositifs de ce type. Ce dispositif de prélèvement d'échantillons liquides comporte un conduit de vidange fortement incliné qui relie le fond du réservoir, dans lequel se trouve l'extrémité d'aspiration de l'aiguille, à la conduite d'arrivée du liquide par le circuit en dépression. Ce conduit de vidange permet d'assurer un renouvellement et une homogénéisation permanente du liquide dans le réservoir et assure la vidange de ce dernier lorsqu'il n'est plus alimenté en liquide.

Par ailleurs, dans le dispositif de prélèvement d'échantillons liquides représenté à la figure 1 du document EP-A-0 078 211, l'embout dans lequel est montée l'aiguille comporte un prolongement tubulaire qui s'étend dans le réservoir au-delà de l'extrémité d'aspiration de l'aiguille. Ce prolongement tubulaire permet d'éviter les turbulences provoquées par l'arrivée dans le réservoir de bulles d'air en provenance du circuit sous vide.

Dans les installations de prélèvement comprenant un banc de prise d'échantillons tel que celui qui est décrit dans les documents FR-A-1 401 298 et FR-A-2 058 751, équipé de dispositifs de prélèvement d'échantillons liquides tels que celui qui est décrit dans le document EP-A-0 078 211 alimentés en liquide par des circuits en dépression tels que celui qui est décrit dans le document EP-A- 0 296 917, chacune des aiguilles des dispositifs de prélèvement d'échantillons liquides met en communication l'intérieur de la boîte à gants du banc de prélèvement et le circuit par lequel le liquide à prélever est amené jusqu'au dispositif de prélèvement comportant cette aiguille. A chaque fois qu'un prélèvement doit être effectué, l'actionnement de la pompe à vide du circuit alimentant le réservoir du dispositif de prélèvement aspire donc au travers de l'aiguille un certain volume d'air présent dans la boîte à gants du banc de prélèvement. Du fait que ce phénomène se reproduit à chaque fois qu'un prélèvement est effectué, il se traduit par une augmentation notable du débit d'air traité dans les circuits en dépression lorsque le nombre de prélèvement devient élevé. Cela allonge le temps de fonctionnement des pompes à chaque prélèvement, ce qui se traduit par un accroissement du coût d'exploitation de l'installation et par une réduction de la durée de vie des pompes.

L'invention a précisément pour objet un dispositif de prélèvement d'échantillons liquides dont la conception originale lui permet de diminuer de façon très sensible le débit de fuite d'air entre la boîte à gants du banc de prélèvement et chacun des circuits en dépression alimentant les réservoirs des dispositifs de prélèvement, de façon à réduire le coût d'exploitation de l'installation et à augmenter sensiblement la durée de vie des pompes à vide.

Conformément à l'invention, ce résultat est obtenu au moyen d'un dispositif de prélèvement d'échantillons liquides, comprenant une aiguille dont une extrémité d'aspiration débouche dans un réservoir apte à être alimenté en liquide par un circuit en dépression, et dont une extrémité pointue est apte à perforer un cruchon en dépression, l'aiguille comportant un chas à proximité de son extrémité pointue, caractérisé par le fait qu'il comprend de plus un manchon coulissant placé autour de l'aiguille et des moyens élastiques maintenant normalement le manchon dans une position d'obturation du chas de l'aiguille, le manchon étant apte à être repoussé à l'encontre des moyens élastiques pour dégager l'extrémité pointue et le chas de l'aiguille.

Du fait que le chas de l'aiguille est normalement obturé par le manchon coulissant, l'actionnement de la pompe à vide du circuit en dépression alimentant le réservoir n'est pratiquement pas influencé par l'air présent dans la boîte à gants du banc de prélèvement. Le remplissage du réservoir s'effectue donc plus rapidement que dans les dispositifs de prélèvement d'échantillons liquides existants. Par conséquent, le coût d'exploitation de l'installation est réduit et la durée de vie des pompes à vide est sensiblement augmentée.

Dans une forme de réalisation préférentielle de l'invention, le chas de l'aiguille débouche latéralement par rapport à un axe longitudinal de cette dernière, de façon à être recouvert par le manchon lorsque ce dernier occupe sa position d'obturation.

Dans cette forme de réalisation préférentielle, le manchon et l'aiguille forment entre eux un jeu calibré, dans la position d'obturation du manchon. Ce jeu calibré crée un débit de fuite réduit entre la boîte à gants du banc de prélèvement et le réservoir alimenté par le circuit en dépression. Ce débit de fuite assure la vidange de l'aiguille, sans pour autant que le débit d'air traité dans le circuit en dépression s'en ressente.

Le dispositif de prélèvement d'échantillons liquides conforme à l'invention comporte en outre, de préférence, un embout porte-aiguille tubulaire, monté coaxialement autour de l'aiguille et délimitant avec cette dernière un espace annulaire dans lequel le manchon est monté coulissant.

Les moyens élastiques comprennent alors un ressort de compression, monté dans l'espace annulaire entre le manchon et une bague solidaire de l'extrémité d'aspiration de l'aiguille, de façon à appliquer le manchon et la bague contre deux épaulements en vis-à-vis formés dans l'embout porte-aiguille.

On décrira à présent, à titre d'exemple non limitatif, une forme de réalisation préférentielle d'un dispositif de prélèvement d'échantillons liquides conforme à l'invention, en se référant aux dessins annexés, dans lesquels :
- la figure 1 est une vue en coupe longitudinale partielle représentant un dispositif de prélèvement d'échantillons liquides selon l'invention, dans la position d'obturation du chas de l'aiguille ; et
- la figure 2 est une vue en coupe longitudinale illustrant à plus grande échelle la partie interchangeable du dispositif de la figure 1, dans la position qu'elle occupe lorsqu'un prélèvement est effectué.

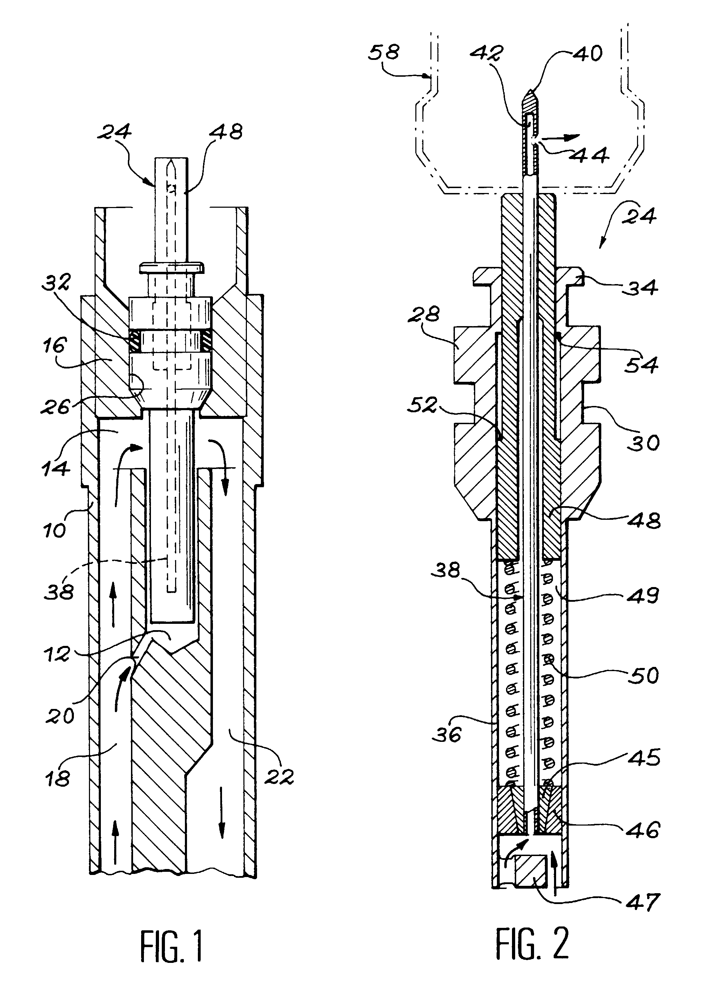

Comme l'illustre la figure 1, le dispositif de prélèvement d'échantillons liquides conforme à l'invention comprend un support 10 qui présente extérieurement la forme d'un cylindre d'axe sensiblement vertical. Ce support 10 est prévu pour être monté de façon étanche dans le fond de la boîte à gants d'un banc de prélèvement, comme l'illustre par exemple le document EP-A-0 078 211.

Le support 10 comporte dans sa partie centrale un réservoir cylindrique 12 disposé selon son axe vertical. Au-dessus du réservoir 12, le support 10 présente une forme tubulaire et délimite intérieurement une chambre 14, fermée vers le haut par un bouchon 16.

Une conduite 18 d'arrivée de liquide chemine verticalement dans le support 10, en un emplacement décalé latéralement par rapport au réservoir 12, et débouche dans la chambre 14 à son extrémité haute. L'extrémité basse de la conduite 18 d'arrivée de liquide est prévue pour être reliée à la partie amont d'un circuit en dépression tel que celui qui est décrit dans le document EP-A-0 296 917. Un conduit de vidange 20, fortement incliné, relie par ailleurs le fond du réservoir 12 à la conduite 18 d'arrivée de liquide.

Une conduite 22 de sortie de liquide, orientée verticalement, est également formée dans le support 10 en un emplacement décalé latéralement par rapport au réservoir 12. Cette conduite 22 de sortie de liquide débouche dans la chambre 14 à son extrémité haute et son extrémité basse est prévue pour être reliée à la partie aval d'un circuit en dépression tel que celui qui est décrit dans le document EP-A-0 296 917.

Le dispositif de prélèvement d'échantillons liquides comprend en outre une partie interchangeable 24, prévue pour être montée dans une ouverture 26, de section circulaire, formée à cet effet dans le bouchon 16, selon l'axe vertical du support 10. Le montage et le démontage de la partie interchangeable 24 sont assurés par des outillages appropriés équipant de façon connue le banc de prélèvement.

Les différents éléments formant la partie interchangeable 24 du dispositif de prélèvement selon l'invention vont à présent être décrits en se référant à la figure 2.

Cette partie interchangeable 24 comporte tout d'abord un embout porte-aiguille 28, de forme généralement tubulaire, prévu pour être emboîté de façon étanche dans l'ouverture 26 du bouchon 16. Cet embout porte-aiguille 28 comporte à cet effet une gorge annulaire 30 dans laquelle est monté un joint d'étanchéité torique 32 (figure 1) normalement en contact étanche avec la paroi de l'ouverture 26.

L'embout porte-aiguille 28 se prolonge vers le haut sous la forme d'une bague de préhension 34 et vers le bas sous la forme d'un prolongement tubulaire 36.

Une aiguille creuse rectiligne 38 est montée coaxialement dans l'embout porte-aiguille 28, de telle sorte que son extrémité basse d'aspiration débouche dans le prolongement tubulaire 36 de l'embout 28, au-dessus de l'extrémité basse de ce prolongement tubulaire.

La partie supérieure de l'aiguille 38 fait saillie vers le haut au-delà de l'extrémité haute de la bague de préhension 34. Cette partie supérieure de l'aiguille 38 est terminée par une extrémité pointue 40 fermant à son extrémité haute le canal central 42 de l'aiguille. A proximité de cette extrémité pointue 40, l'aiguille creuse 38 comporte un chas 44, par lequel le canal central 42 de l'aiguille débouche latéralement dans la boîte à gants du banc de prélèvement, au-dessus du bouchon 16 du dispositif.

Une bague d'appui 45 tronconique est fixée, par exemple par soudure, sur la partie inférieure de l'aiguille creuse 38. Cette bague d'appui tronconique 45 repose normalement sur une bague d'appui complémentaire 46 fixée dans le prolongement tubulaire 36 de l'embout porte-aiguille 28, par exemple par soudure. La bague d'appui 46 forme ainsi un épaulement qui sert à la fois à centrer l'aiguille creuse 38 dans l'embout porte-aiguille 28 et à positionner l'aiguille verticalement dans cet embout.

Un dispositif déflecteur 47 est monté dans la partie basse du prolongement tubulaire 36 de l'embout porte-aiguille 28, immédiatement en dessous de la bague d'appui 46 et de l'extrémité d'aspiration de l'aiguille 38. Ce dispositif déflecteur permet d'éviter les turbulences à proximité de l'extrémité basse d'aspiration de l'aiguille creuse 38.

Au-dessus des bagues d'appui 45 et 46, l'embout porte-aiguille 28 et l'aiguille creuse 38 délimitent entre eux un espace annulaire 49 dans lequel sont placés, l'un au-dessus de l'autre, un manchon coulissant 48 et un ressort hélicoïdal de compression 50. Ce ressort 50 constitue un moyen élastique de rappel du manchon coulissant 48 vers sa position normale d'obturation du chas 44 de l'aiguille 38.

De façon plus précise, le manchon 48 est monté dans l'embout porte-aiguille 28 de façon à coulisser librement dans ce dernier selon une direction verticale. Le ressort hélicoïdal de compression 50 est placé autour de l'aiguille 38, de telle sorte que son extrémité basse soit en appui sur la bague d'appui tronconique 45 liée à l'aiguille et que son extrémité haute soit en appui sur la face d'extrémité basse du manchon coulissant 48. Le ressort 50 maintient ainsi les bagues 45 et 46 en appui l'une contre l'autre et sollicite le manchon coulissant 48 vers sa position normale d'obturation du chas 44 de l'aiguille. Cette position normale d'obturation est déterminée par la venue en appui d'un épaulement 52 formé sur le manchon coulissant 48 contre un épaulement 54 formé dans l'embout porte-aiguille 28.

Dans cette position normale d'obturation, la partie haute du manchon coulissant 48 entoure totalement la partie supérieure de l'aiguille creuse 38, qui fait saillie au-dessus de l'embout porte-aiguille 28, comme on l'a illustré sur la figure 1, de telle sorte que le chas 44 de l'aiguille débouche dans le manchon.

Il est à noter que, dans cette position normale d'obturation occupée par le manchon coulissant 48 sous l'action du ressort 50, il existe normalement entre l'aiguille 38 et le manchon un jeu calibré grâce auquel un débit de fuite réduit par exemple de l'ordre de 0,05 Nm³/h reste possible entre la boîte à gants du banc de prélèvement et le réservoir 12, au travers de l'aiguille creuse. Ce débit de fuite est suffisant pour permettre la vidange de l'aiguille 38 et du réservoir 12 lorsque l'alimentation du réservoir 12 est arrêtée c'est-à-dire lorsque la pompe à vide du circuit en dépression raccordé sur les conduites 18 et 22 (figure 1) est arrêtée. Toutefois, ce débit de fuite est suffisamment réduit pour que la quantité d'air aspirée par la pompe à vide à partir de la boîte à gants soit négligeable.

Comme on l'a représenté sur la figure 2, lorsqu'un cruchon 58 (illustré en traits mixtes) est appliqué contre la face supérieure du manchon coulissant 48, et déplacé vers le bas, le manchon 48 est repoussé vers le bas à l'encontre du ressort 50, de telle sorte que l'extrémité pointue 40 de l'aiguille 38 se trouve dégagée et perfore l'opercule du cruchon 58. Lorsque le manchon 48 est suffisamment repoussé pour que le chas 44 de l'aiguille 38 se trouve à l'intérieur du cruchon 58 (figure 2), la dépression qui règne dans ce dernier assure automatiquement l'aspiration d'un échantillon de liquide présent dans le réservoir 12, au travers de l'aiguille creuse 38.

La description qui précède montre que le dispositif de prélèvement d'échantillons liquides selon l'invention permet de réaliser les prélèvements dans des conditions comparables à celles du dispositif de l'art antérieur, tout en évitant que les pompes à vide du circuit en dépression alimentant le réservoir 12 n'aspirent inutilement l'air présent dans la boîte à gants du banc de prélèvement. La vidange de l'aiguille et du réservoir reste cependant assurée dans des conditions satisfaisantes.

## Revendications

1. Dispositif de prélèvement d'échantillons liquides, comprenant une aiguille (38) dont une extrémité d'aspiration débouche dans un réservoir (12) apte à être alimenté en liquide par un circuit en dépression, et dont une extrémité pointue (40) est apte à perforer un cruchon (58) en dépression, l'aiguille (38) comportant un chas (44) à proximité de son extrémité pointue, caractérisé par le fait qu'il comprend de plus un manchon coulissant (48) placé autour de l'aiguille (38) et des moyens élastiques (50) maintenant normalement le manchon dans une position d'obturation du chas (44) de l'aiguille, le manchon (48) étant apte à être repoussé à l'encontre des moyens élastiques (50) pour dégager l'extrémité pointue (40) et le chas (44) de l'aiguille (38).

2. Dispositif selon la revendication 1, caractérisé par le fait que le chas (44) de l'aiguille (38) débouche latéralement par rapport à un axe longitudinal de cette dernière, de façon à être recouvert par le manchon (48) lorsque ce dernier occupe sa position d'obturation.

3. Dispositif selon la revendication 2, caractérisé par le fait que le manchon (48) et l'aiguille (38) forment entre eux un jeu calibré, dans la position d'obturation du manchon.

4. Dispositif selon l'une quelconque des revendications précédentes, caractérisé par le fait qu'il comprend de plus un embout porte-aiguille tubulaire (28), monté coaxialement autour de l'aiguille (38) et délimitant avec cette dernière un espace annulaire (49) dans lequel est monté le manchon (48).

5. Dispositif selon la revendication 4, caractérisé par le fait que les moyens élastiques comprennent un ressort de compression (50), monté dans ledit espace annulaire (49) entre le manchon (48) et une bague (45) solidaire de l'extrémité d'aspiration de l'aiguille (38), de façon à appliquer le manchon et la bague contre deux épaulements en vis-à-vis (46,54) formés dans l'embout porte-aiguille (28).

6. Dispositif selon l'une quelconque des revendications 4 et 5, caractérisé par le fait qu'un dispositif déflecteur (47) est monté dans l'embout porte-aiguille (28), au-delà de l'extrémité d'aspiration de l'aiguille (38).
